# EUROPEAN PATENT APPLICATION

(11) **EP 4 071 231 A1**
(43) Date of publication of application: **12.10.2022**
(21) Application number: 20896678.8
(22) Date of filing: 02.12.2020
(51) Int. Cl.: C12M 1/06, B01F 13/08, B01F 15/00

(54) **BIOREACTOR AGITATOR**

(30) Priority: 02.12.2019 KR 20190158153
(71) Applicant: Kim, Doo-Hyun, Seoul 06503 (KR)
(72) Inventor: Kim, Doo-Hyun, Seoul 06503 (KR)
(74) Representative: Hübner, Gerd
(86) International application number: PCT/KR2020/017434
(87) International publication number: WO 2021/112545

(57) **Abstract**

The present invention relates to a bioreactor agitator. According to one embodiment of the present invention a drive coupling part is coupled to an impeller part through the drive coupling part, and the rotary shaft coupled to the impeller by means of magnetic force is provided to be adjustable in height by means of an up-and-down transfer part, such that the entire interior of the housing part can be effectively stirred.

## Description

### Technical Field

The present invention relates to a bioreaction agitator.

This application claims priority based on Korean Patent Application No. 10-2019-0158153 filed on December 2, 2019, and all contents described in the specification and drawings of the application are incorporated herein by reference.

### Background Art

Currently, various types of bioreactors including a cell incubator are widely used in a great range of fields including medicine, pharmaceuticals, biotechnology, etc., for the purposes of production of reagents or vaccines, development of new drugs, and treatment and researches using stem cells.

A bioreactor refers to a system that artificially reproduces biochemical reaction processes such as decomposition, synthesis or chemical transformation of substances, etc., in the body of an organism, and is also called a bio-reaction device.

Therefore, depending on the details of various operations performed in the bioreactor, different conditions such as nutrients, temperature, humidity, PH, oxygen and carbon dioxide that are suitable for the inside of a container, in which mixing of substances or culturing of cells actually occurs, should be maintained for a predetermined period.

On the other hand, Korean Patent Laid-Open Publication No. 10-2010-0083524 (laid-opened on July 22, 2010), as a related art thereto, disclosed "a method for construction of a stirrer in a longitudinal bioreactor and a bioreactor comprising one vertical stirrer".

The above Korean patent laid-open publication has an advantage of promoting the biochemical reaction of a sample inside the bioreactor because a stirring blade is connected to a driving unit through an impeller shaft to rotate by a driving force of the driving unit.

However, the above Korean patent laid-open publication has a disadvantage in that the entire inside of the bioreactor cannot be efficiently stirred because the stirring blade is fixed at a predetermined position inside the bioreactor through the impeller shaft, hence creating a blind spot inside the bioreactor.

### Disclosure

### Technical Problem

As the technical problem to be achieved by the present invention, an object of the present invention is to provide a bioreaction agitator capable of efficiently stirring the entire inside of a housing unit, which includes: a drive coupling unit formed in an axial direction inside the housing unit; and a rotational shaft coupled to an impeller unit through the drive coupling unit so as to reduce a blind spot in a lower portion of the housing unit as much as possible, wherein the rotational shaft is coupled to an impeller by magnetic force and is provided to be adjustable in height through an up-and-down (or vertical) transfer unit.

The object of the present invention is not limited thereto, and other objects not mentioned herein will be clearly understood by persons having ordinary skill in the art ("those skilled in the art") from the following description.

### Technical Solution

In order to achieve the above purposes, one embodiment of the present invention provides a bioreaction agitator, including: a housing unit in which a biochemical reaction of a sample occurs inside, and a drive coupling unit is formed such that it is recessed toward the inside from one side or perforates from one side toward the other side; a driving unit provided inside the drive coupling unit, which is provided with a driving magnetic unit rotated by a driving force supplied from a driving device; and a stirring unit disposed along a periphery of the drive coupling unit inside the housing unit, which includes a driven magnetic unit rotated by magnetic force of the driving magnetic unit and an impeller unit connected to the driven magnetic unit.

Further, with regard to the bioreaction agitator provided by the present invention, the driven magnetic unit may include: a rotary case provided with the impeller unit on the outside thereof, which surrounds the drive coupling unit while being spaced apart from the same at a predetermined interval; and a driven magnet provided in the rotary case, which rotates together with the rotary case by the magnetic force of the driving magnetic unit.

Further, with regard to the bioreaction agitator provided by the present invention, the driven magnetic unit may further include a friction-reducing member, which is rotatably provided on an inner peripheral surface of the rotary case and, when rotating the rotary case, is rotated in close contact with the drive coupling unit so as to reduce friction with the drive coupling unit.

With regard to the bioreaction agitator provided by the present invention, the driven magnet may further include: a magnet coupling member which is fixedly coupled to the inner peripheral surface of the rotary case; and a friction-reducing magnet rotatably coupled to the magnet coupling member, which rotates along the periphery of the drive coupling unit by the magnetic force of the driving magnetic unit and, when rotating the rotary case, is in close contact with the drive coupling unit and rotated with respect to the magnet coupling member so as to reduce friction with the drive coupling unit.

### Advantageous effects

According to an embodiment of the present invention, a drive coupling unit is axially formed inside a housing unit and a rotational shaft is coupled to an impeller unit through the drive coupling unit, thereby reducing a blind spot on a lower portion of the housing unit as much as possible. Further, the rotational shaft coupled to an impeller by magnetic force is provided to be adjustable in height through a vertical transfer unit, thereby attaining effects of efficiently stirring the entire inside of the housing unit.

### Description of Drawings

FIG. 1 is a cross-sectional view of a bioreaction agitator according to an embodiment of the present invention.
FIG. 2 is an exploded perspective view of the bioreaction agitator shown in FIG. 1.
FIG. 3 is a cross-sectional view and a partially enlarged view for explaining an internal structure of the bioreaction agitator shown in FIG. 1.
FIG. 4 is an exploded perspective view showing a stirring unit according to another embodiment of the present invention, which is separated from a lower housing shown in FIG. 1.
FIG 5 is a cross-sectional view and a partially enlarged view showing the bioreaction agitator provided with a driven magnetic unit according to another embodiment of the present invention.
FIG. 6 is a cross-sectional view of a bioreaction agitator according to another embodiment of the present invention.
FIG. 7 is a cross-sectional view of a bioreaction agitator according to a further embodiment of the present invention.

### Description of numeral symbols

10, 20, 30: Bioreaction agitator
101: Housing unit 103: Upper housing
105: Lower housing 201: Drive coupling unit
203: Driving unit 205: Driven magnetic unit
207: Impeller unit 209: Stirring unit
211: Rotational shaft 213: Rotary case
215: Rotational coupling hole 217: Friction-reducing member
301: Driving magnetic unit 303: Inner space
305: Driving magnet 307: Driven magnet
309: Gap 311: Fixed coupling unit
313 Bearing unit 401: Impeller unit
403: Rotary case 501: Driven magnet
503: Magnet coupling member 505: Friction-reducing magnet
507: Rotational projection 601: Drive coupling unit
603: Housing unit 605: Driving magnetic unit
607: Driving unit 609: Driven magnetic unit
611: Impeller unit 613: Stirring unit
615: Upper housing 617: Lower housing
619: Inner space 621: Rotary case
623: Driven magnet 625: Friction-reducing member
627: Upper magnet 629: Lower magnet
631: Rotational shaft 701: Upper guide
703: Lower guide 705: Gap

### Detailed Description of Preferred Embodiments of Invention

Hereinafter, some embodiments of the present invention will be described in detail with reference to exemplary drawings. With respect to addition of reference numerals to the components of each drawing, it should be noted that the same components are given the same reference numerals as much as possible even though they are indicated on different drawings. Further, in describing the present invention, if it is determined that a detailed description of a related known configuration or function may obscure the gist of the present invention, the detailed description thereof will be omitted.

Further, in describing the components of the present invention, terms such as first, second, A, B, (a), (b), etc. may be used. These terms are only for distinguishing the components from other components, and the essence, sequence and/or order of the components are not limited by the terms. When a component is described as being "bound", "coupled" or "connected" to another component, the component may be directly bound or connected to the other component, but it should be understood that another component (s) may be "bound", "coupled" or "connected" between two components.

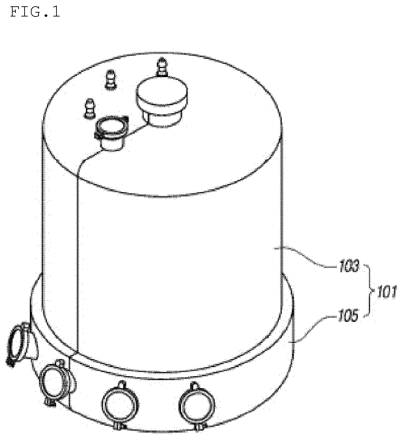

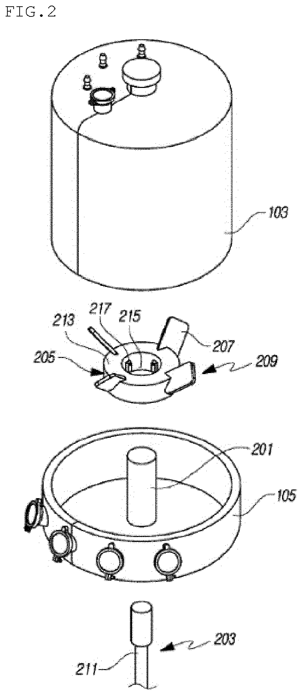

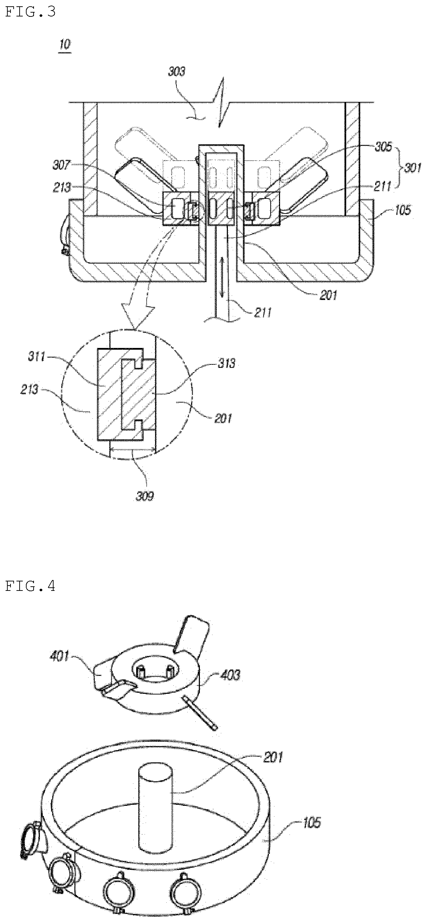

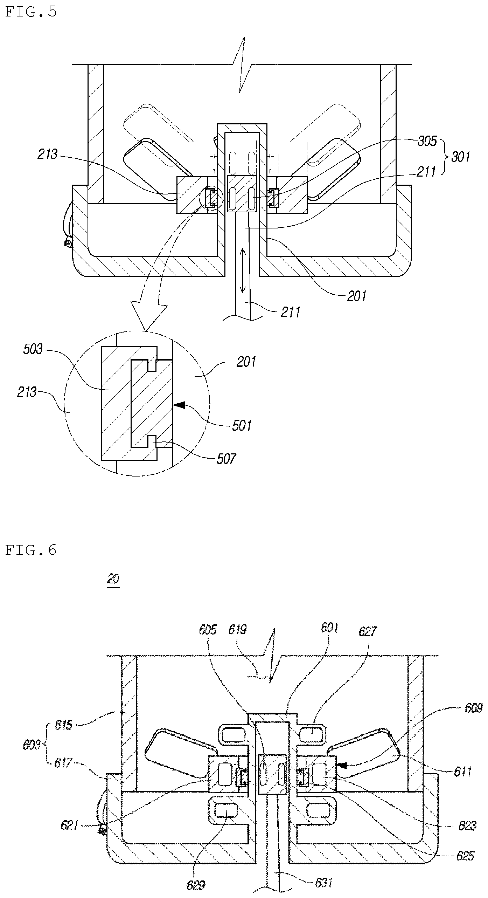

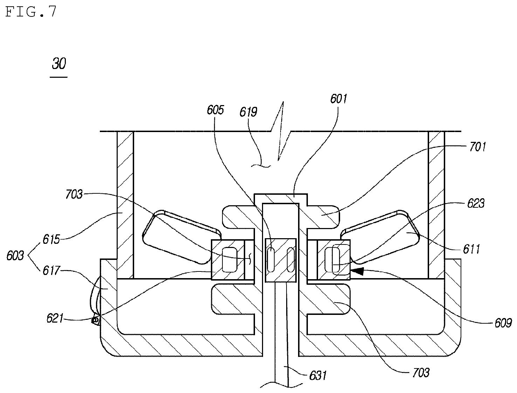

FIG. 1 is a cross-sectional view of a bioreaction agitator according to an embodiment of the present invention. FIG. 2 is an exploded perspective view of the bioreaction agitator shown in FIG. 1. FIG. 3 is a cross-sectional view and a partially enlarged view for explaining an internal structure of the bioreaction agitator shown in FIG. 1. FIG. 4 is an exploded perspective view showing a stirring unit according to another embodiment of the present invention, which is separated from a lower housing shown in FIG. 1. FIG 5 is a cross-sectional view and a partially enlarged view showing the bioreaction agitator provided with a driven magnetic unit according to another embodiment of the present invention. FIG. 6 is a cross-sectional view of a bioreaction agitator according to another embodiment of the present invention. FIG. 7 is a cross-sectional view of a bioreaction agitator according to a further embodiment of the present invention.

As shown in these figures, the bioreaction agitator according to an embodiment of the present invention may include: a housing unit 101 in which a biochemical reaction of a sample occurs inside, and a drive coupling unit 201 is formed such that it is recessed toward the inside from one side or perforates from one side toward the other side; a driving unit 203 provided inside the drive coupling unit 201, which is provided with a driving magnetic unit 301 rotated by a driving force supplied from a driving device; and a stirring unit 209 disposed along a periphery of the drive coupling unit 201 inside the housing unit 101, which includes a driven magnetic unit 205 rotated by magnetic force of the driving magnetic unit 301 and an impeller unit 207 connected to the driven magnetic unit 205.

The housing unit 101 may include an upper housing 103 provided on an upper portion thereof and a lower housing 103 coupled to the upper housing 103 to form an inner space 303, whereby the biochemical reaction of a sample occurs inside the housing unit.

Herein, the sample is accommodated in the inner space 303, and stirring is conducted so as to actively perform the biochemical reaction of the sample.

Further, the housing unit 101 has the drive coupling unit 201 that may be recessed toward the inside from one side or perforate from one side to the other side. In the drawings, an example of the drive coupling unit 201 recessed toward the inner space 303 from the lower housing 105 is illustrated.

The drive coupling unit 201 may be formed in a cylindrical groove shape on the lower housing 105, and may include a rotational shaft 211 rotatably coupled therein and a rotary case 213 rotatably coupled on the outside thereof.

That is, the drive coupling unit 201 may protrude in a cylindrical form toward an upper side of the lower housing 105 and serve as a standard axis enabling rotation of the rotary case 213.

Meanwhile, in the case of the bioreaction agitator 10, after an operation such as cell culture is completed, the bioreaction agitator 10 can be re-used through a number of procedures, for example, complete removal of residues on the inner space 303 of the housing unit 101 and performing sterilization again. Therefore, for reasons of hygiene, etc., the housing unit 101 may also be provided in the form of a disposable film having flexibility.

Following this, the driving unit 203 may include a driving device (not shown) and a driving magnetic unit 303 which is rotated inside the drive coupling unit 201 by a driving force supplied from the driving device.

Although the driving device is not illustrated in the drawings, it may be provided in the form of a motor rotating by an external power supply and may be located on a base unit (not shown) to which a vertical transfer unit (not shown) is coupled to be adjustable in height.

When describing an example of the structure of the driving magnetic unit in more detail, the driving magnetic unit 301 may be provided inside the drive coupling unit 201 and rotated by a driving force supplied from the driving device, and may include: a rotational shaft 211 provided to be adjustable in height through the vertical transfer unit; and a driving magnet 305 provided on the rotational shaft 211, which rotates the stirring unit 209 with respect to the drive coupling unit 201 when rotating the rotational shaft 211.

The rotational shaft 211 is coupled to the driving device and rotated inside the drive coupling unit 201, and may be coupled to be spaced apart from an inner peripheral surface of the drive coupling unit 201 at a predetermined interval, thereby preventing friction with the drive coupling unit 201 during rotation.

That is, the rotational shaft 211 is formed to have a smaller diameter than a diameter of the drive coupling unit 201 and may be coupled to be spaced apart from the drive coupling unit 201 at a predetermined interval when coupled to the drive coupling unit 201, thereby preventing friction with the drive coupling unit 201 during rotation.

The driving magnet 301 may be provided in plural and arranged in a circumferential direction of the rotational shaft 211 at equal intervals on an upper end of the rotational shaft 211.

Further, the driving magnet 301 has polarity on a side facing the driven magnetic unit 205, which is different from that of a driven magnet 307 to be described later, so as to be coupled to the driven magnetic unit 205 through magnetic force.

As described above, the driving magnetic unit 301 is height-adjustable by the vertical transfer unit to thus adjust a height of the stirring unit 209, so that the stirring unit 209 can agitate the entire inner space 303.

Subsequently, the stirring unit 209 may include the driven magnetic unit 205 coupled to the outside of the drive coupling unit 201, as well as an impeller unit 209 provided on the outside of the driven magnetic unit 205.

Specifically, the driven magnetic unit 205 may include a rotary case 213 which is provided with the impeller unit 207 on the outside, and surrounds the drive coupling unit 201 while being spaced apart from the same at a predetermined interval; and a driven magnet 307 provided on the rotary case 213, which rotates together with the rotary case 213 by magnetic force of the driving magnetic unit 301.

The rotary case 213 is rotatably coupled to the drive coupling unit 201, and more specifically, the rotary case 213 may have a rotational coupling hole 215 formed in the center thereof to perforate in an axial direction, whereby the rotary case is inserted to the outside of the drive coupling unit 201 through the rotational coupling hole 215.

Herein, the rotational coupling hole 215 may be formed to have a larger diameter than a diameter of the drive coupling unit 201. As such, owing to a structure of the rotational coupling hole 215 with a larger diameter compared to a diameter of the drive coupling unit 201, the rotary case 213 may be spaced apart from the drive coupling unit 201 at a predetermined interval when inserted into the drive coupling unit 201, thereby preventing friction with an outer periphery of the drive coupling unit 201 during rotation.

In other words, since the rotary case 213 has a rotational coupling hole 215 larger than the diameter of the drive coupling unit 201, a gap 309 may occur between the rotary case and the drive coupling unit 201 when inserted into the drive coupling unit 201. As such, owing to a structure in that, when the rotational coupling hole 215 is coupled to the drive coupling unit 201, a gap 309 is formed to prevent friction with the drive coupling unit 201 during rotation, the present invention may prevent a residue, which is generated by friction between the rotary case 213 and the drive coupling unit 201 when rotating the stirring unit 209, from penetrating into the sample.

Further, the rotary case 213 is coupled to the drive coupling unit 201 in a height-adjustable manner. More specifically, since the rotational shaft 211 and the driving magnet 305 are adjustable in height, the rotary case 213 may be height-adjustable by magnetic force of the driven magnet 307 whose polarity is different from that of the driving magnet 305.

At this time, the impeller unit 207 provided on the outside of the rotary case 213 may also be height-adjustable along with the rotary case 213, thereby agitating the entire inner space 303.

In addition, the rotary case 213 may be detachably coupled to the drive coupling unit 201.

In other words, owing to a structure in that the rotary case 213 is detachably coupled to the drive coupling unit 201, the present invention may replace only the rotary case 213 and the impeller unit 207 when the rotary case 213 or impeller unit 207 is damaged or destroyed. Further, as shown in FIG. 4, the above rotary case and impeller unit may be replaced with an alternative rotary case 213, in which an impeller unit 401 in a different shape is arranged at a different angle as needed, thereby more efficiently agitating the inner space 303.

The driven magnet 307 is provided in the rotary case 213, and more specifically, the driven magnet 307 may be provided in plural in a circumferential direction at equal intervals inside the rotary case.

The driven magnet 307 has polarity on a side facing the driving magnet 301, which is different from that of the driven magnet 301 so as to be coupled to the driving magnet 301 through magnetic force. As such, owing to a structure in that the driven magnet has polarity different from that of the driving magnet 301, the driven magnet may rotate together with the driving magnet 301 by magnetic force when rotating the driving magnet 301.

On the other hand, referring to FIG. 5, a driven magnet 501 according to another embodiment of the present invention may include: a magnet coupling member 503 fixedly coupled to an inner peripheral surface of the rotary case 213; a friction-reducing magnet 505 rotatably coupled to the magnet coupling member 503, which rotates along a periphery of the drive coupling unit 201 by magnetic force of the driving magnetic unit 301 and, when rotating the rotary case 213, is in close contact with the drive coupling unit 201 and rotated with respect to the magnet coupling member 503, thereby reducing friction with the drive coupling unit 201.

Specifically, the magnet coupling member 503 is fixedly coupled to the inner peripheral surface of the rotary case 21 and, when rotating the rotary case 213, rotates along a periphery of the fixed coupling unit 201 together with the rotary case 213, and may include rotational projections protruding inward in an axial direction at both ends so as to rotate the friction-reducing magnet 505.

The friction-reducing magnet 505 is approximately formed in a cylindrical shape, and may include rotational coupling grooves recessed inward in the axial direction at both ends, which are rotatably coupled to the rotational projections 507, and thus, are rotatably coupled to the magnet coupling member 503.

The friction-reducing magnet 505 is configured to have polarity different from that of the driving magnet 305 and, when rotating the rotational shaft 211, may rotate along the periphery of the drive coupling unit 201 by magnetic force together with the driving magnet 305 so as to rotate the magnet coupling member 503 and the rotary case 213.

Further, the friction-reducing magnet 505 is rotatably coupled to the magnet coupling member 503 and, when it is in close contact with the drive coupling unit 201 and rotates along the periphery of the drive coupling unit 201, may rotate with respect to the rotational projection 507 so as to reduce friction between the rotary case 213 and the drive coupling unit 201.

Meanwhile, referring to FIGS. 2 and 3, the driven magnet 307 according to an embodiment of the present invention is rotatably provided on an inner peripheral surface of the rotary case 213 and, when rotating the rotary case 201, may be rotated in close contact with the drive coupling unit 201 so as to reduce friction with the drive coupling unit 201.

The friction-reducing member 217 is disposed in a gap 309 between the rotary case 213 and the drive coupling unit 201. When describing an example of the structure of the friction-reducing member 217 in more detail, the friction-reducing member 217 is fixedly coupled to an inner peripheral surface of the rotary case 213, and may include: a fixed coupling unit 311 which rotates along the periphery of the drive coupling unit 201 together with the rotary case 213 when rotating the rotary case 213; and a bearing unit 313 rotatably coupled to the fixed coupling unit 311, which is in close contact with the drive coupling unit 201 and rotated with respect to the fixed coupling unit 311 when rotating the rotary case 213, thereby reducing friction with the drive coupling unit 201.

Herein, the bearing unit 313 is approximately formed in a cylindrical shape, both ends of which are axially coupled to the fixed coupling unit 311 so as to be rotatably coupled to the fixed coupling unit 311.

Meanwhile, referring to FIG. 6, the bioreaction agitator 20 according to another embodiment of the present invention may include: a housing unit 603 in which a biochemical reaction of a sample occurs inside, and a drive coupling unit 601 is formed such that it is recessed toward the inside from one side or perforates from one side toward the other side; a driving unit 607 provided inside the drive coupling unit 601, which is provided with a driving magnetic unit 605 rotated by a driving force supplied from a driving device; and a stirring unit 613 disposed along a periphery of the drive coupling unit 601 inside the housing unit 603, which includes a driven magnetic unit 609 rotated by magnetic force of the driving magnetic unit 603 and an impeller unit 611 connected to the driven magnetic unit 609.

In this regard, the driving unit 607 and the stirring unit 613 in the bioreaction agitator 20 according to another embodiment of the present invention are substantially configured in the same way as the driving unit 203 and the stirring unit 209 in the bioreaction agitator 10 according to the previous embodiment of the present invention.

Further, the driven magnetic unit 609 according to another embodiment of the present invention may include: a rotary case 621 which is provided with an impeller unit 611 on the outside thereof in the same manner as the driven magnetic unit 205 according to the previous embodiment of the present invention, and surrounds the drive coupling unit 601 while being spaced apart from the same at a predetermined interval; a driven magnet 623 provided in the rotary case 621, which rotates together with the rotary case 621 by magnetic force of the driving magnetic unit 605; and a friction-reducing member 625 rotatably provided on an inner peripheral surface of the rotary case 621, which is rotated in close contact with the drive coupling unit 601 so as to reduce friction with the drive coupling unit 601 when rotating the rotary case 621.

Meanwhile, the housing unit 603 may include an upper housing 615 and a lower housing 617.

Further, the housing unit 603 includes the drive coupling 601 which is recessed inward from one side or perforates from one side toward the other side. In the drawings, an example of the drive coupling unit 601 that is recessed toward an inner space 619 from the lower housing 617 was illustrated.

The drive coupling unit 601 is recessed toward the inner space 619 from the lower housing 617, so that the rotary case 621 provided with the impeller unit 611 on the outside thereof is rotatably coupled.

Further, the drive coupling unit 601 may include an upper magnet 627 provided on an upper portion of the driven magnet 623 and a lower magnet 629 provided on a lower portion thereof.

The upper magnet 627 is provided with the same polarity as that of the driven magnet 623 of the driven magnetic unit 609, so as to supply a repulsive force to the driven magnet 609 downward in the height direction.

A plurality of the upper magnets 627 may be arranged at equal intervals in a circumferential direction of the drive coupling unit 601, otherwise, may be continuously arranged in the circumferential direction of the drive coupling unit 601 to thus supply a repulsive force, thereby moving the driven magnet 623 downward.

Further, the lower magnet 629 may be provided with the same polarity as the driven magnet 623 of the driven magnetic unit 609 to thus supply a repulsive force to the driven magnetic unit 609 upward in the height direction.

Similarly to the upper magnet 627, a plurality of the lower magnets 629 may be arranged at equal intervals in the circumferential direction of the drive coupling unit 601, otherwise, may be continuously arrange in the circumferential direction of the drive coupling unit 601, to thus supply a repulsive force, thereby moving the driven magnet 623 upward.

As such, according to a structure in that the upper magnet 627 to supply a repulsive force to the driven magnet 623 downward in the height direction and the lower magnet 629 to supply a repulsive force upward in the height direction are provided, the stirring unit 613 according to another embodiment of the present invention is height-fixed by magnetic force at a predetermined position of the drive coupling 601 so that friction between the stirring unit 613 and the housing unit 603 can be maximally reduced.

On the other hand, referring to FIG. 7, the bioreaction agitator 30 according to a further embodiment of the present invention may have the same structure as the bioreaction agitator 20 according to another embodiment of the present invention except for some configurations.

For example, an upper guide 701 may be provided at the position of the upper magnet 627, while a lower guide 703 may be provided at the position of the lower magnet 629.

Further, since the friction-reducing member 625 provided between the drive coupling unit 601 and the rotary case 621 is removed, a gap 703 may occur between the drive coupling unit 601 and the rotary case 621.

At this time, the upper guide 701 and the lower guide 703 protrude in a radial direction from the upper portion and lower portion of the drive coupling unit 601, respectively, and thus may rotatably constrain the driven magnetic unit 609 during driving, thereby preventing the driven magnetic unit 609 from escaping.

As such, according to the further embodiment of the present invention, the gap 703 is formed between the drive coupling unit 601 and the rotary case 621 so that friction occurring during driving can be maximally reduced. Further, it is possible to prevent the driven magnetic unit 609 from escaping the drive coupling unit 601 by means of the upper guide 701 and the lower guide 703.

As described above, according to the embodiments of the present invention, the drive coupling units 201 and 601 are formed in the axial direction in the housing units 101 and 603, respectively, and the rotational shafts 211 and 631 are connected to the impeller units 207 and 611 through the drive coupling units 201 and 601, respectively, whereby a blind spot in the lower portion of each of the housing units 101 and 603 is maximally reduced. Further, the rotational shafts 211 and 631 coupled to the impeller units 207 and 611, respectively, by magnetic force may be provided to be adjustable in height through the vertical transfer unit, thereby attaining effects of efficiently agitating the entire inside of the housing units 101 and 603.

In the above, preferred embodiments of the present invention have been illustrated and described, however, the present invention is not limited to such specific and preferred embodiments as described above. Therefore, anyone skilled in the technical field to which the present invention pertains would of course implement various modifications without departing from the gist of the present invention as claimed in the appended claims, and such alterations are within the scope of the claims.

## Claims

1. A bioreaction agitator, comprising:
a housing unit in which a biochemical reaction of a sample occurs inside thereof, and a drive coupling unit is formed such that it is recessed toward the inside there of from one side or perforates from one side toward another side;
a driving unit provided inside the drive coupling unit, which is provided with a driving magnetic unit rotated by a driving force supplied from a driving device; and
a stirring unit disposed along a periphery of the drive coupling unit inside the housing unit, which includes a driven magnetic unit rotated by magnetic force of the driving magnetic unit and an impeller unit connected to the driven magnetic unit.

2. The bioreaction agitator according to claim 1, wherein the driven magnetic unit includes:
a rotary case provided with the impeller unit on the outside thereof, which surrounds the drive coupling unit while being spaced apart from the same at a predetermined interval; and
a driven magnet provided in the rotary case, which rotates together with the rotary case by the magnetic force of the driving magnetic unit.

3. The bioreaction agitator according to claim 2, wherein the driven magnetic unit is arranged in plural in a circumferential direction at equal intervals inside the rotary case.

4. The bioreaction agitator according to claim 2, wherein the driven magnetic unit further includes,
a friction-reducing member, which is rotatably provided on an inner peripheral surface of the rotary case and, when rotating the rotary case, is rotated in close contact with the drive coupling unit so as to reduce friction with the drive coupling unit.

5. The bioreaction agitator according to claim 2, wherein the driven magnet includes:
a magnet coupling member which is fixedly coupled to an inner peripheral surface of the rotary case; and
a friction-reducing magnet rotatably coupled to the magnet coupling member, which rotates along the periphery of the drive coupling unit by the magnetic force of the driving magnetic unit and, when rotating the rotary case, is in close contact with the drive coupling unit and rotated with respect to the magnet coupling member, so as to reduce friction with the drive coupling unit.

6. The bioreaction agitator according to claim 2, wherein the drive coupling unit further includes,
a lower magnet disposed on a lower portion of the driven magnetic unit, which is provided with the same polarity as the driven magnetic unit to thus supply a repulsive force to the driven magnetic unit in a height direction.

7. The bioreaction agitator according to any one of claims 1 to 6, wherein the driving magnetic unit includes:
a rotational shaft provided inside the drive coupling unit, which is provided to be adjustable in height through a vertical transfer unit while rotating by a driving force supplied from the driving device; and
a driving magnet provided on the rotational shaft, which rotates the stirring unit with respect to the drive coupling unit when rotating the rotational shaft.
